(19) Europäisches Patentamt
European Patent Office
Office européen des brevets

(11) Veröffentlichungsnummer: **0 193 774**
**B1**

(12) **EUROPÄISCHE PATENTSCHRIFT**

(45) Veröffentlichungstag der Patentschrift: **01.06.88**

(21) Anmeldenummer: **86101986.7**

(22) Anmeldetag: **17.02.86**

(51) Int. Cl.⁴: **C 07 C 149/26**, B 01 J 31/22, C 23 F 11/16, C 09 K 19/40, C 09 K 15/10, B 41 M 5/18, B 41 M 5/26, G 03 C 1/72

(54) **Campherdithiolen-Komplexe und deren Verwendung.**

(30) Priorität: **20.02.85 DE 3505750**

(43) Veröffentlichungstag der Anmeldung: **10.09.86 Patentblatt 86/37**

(45) Bekanntmachung des Hinweises auf die Patenterteilung: **01.06.88 Patentblatt 88/22**

(84) Benannte Vertragsstaaten: **CH DE FR GB IT LI NL**

(56) Entgegenhaltungen:
**DE - A - 2 456 075**
**DE - A - 3 319 738**
**GB - A - 1 263 910**
**US - A - 4 325 793**

**Die Akte enthält technische Angaben, die nach dem Eingang der Anmeldung eingereicht wurden und die nicht in dieser Patentschrift enthalten sind.**

(73) Patentinhaber: **BASF Aktiengesellschaft, Carl-Bosch-Strasse 38, D-6700 Ludwigshafen (DE)**

(72) Erfinder: **Schrott, Wolfgang, Dr., Brüsseler Ring 45, D-6700 Ludwigshafen (DE)**
Erfinder: **Neumann, Peter, Dr., Franz-Schubert-Strasse 1, D-6908 Wiesloch (DE)**
Erfinder: **Albert, Bernhard, Dr., Riethburgstrasse 13, D-6701 Maxdorf (DE)**

**EP 0 193 774 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

ACTORUM AG

**Beschreibung**

Aus der Literatur ist eine grosse Zahl von binären Dithiolen-Komplexen, die der allgemeinen Formel

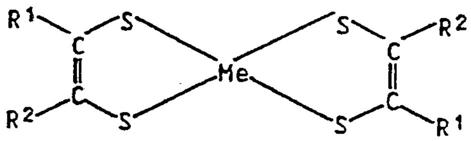

(I)

entsprechen, bekannt, worin die Reste $R^1$ und $R^2$ ebenso wie das Zentralmetall Me in grosser Breite variieren. $R^1$, $R^2$ können ausser Wasserstoff, Alkyl, gegebenenfalls substituiertes Aryl, CN, $CF_3$, SR und andere Reste sein. Neben den binären Komplexen sind auch trinäre Dithiolen-Komplexe der Formel

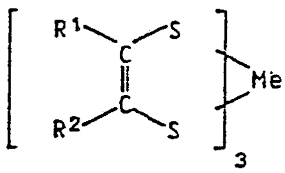

(II)

bekannt. Eine Zusammenstellung findet sich in: J.A. McCLEVERTY, Progr. Inorg. Chem. *10*, S. 49-221 (1968) und G.N. SCHRAUZER, Acc. Chem. Res. *2*, S. 72-80 (1969).

So werden in der GB-A-1 263 910 Komplexe der Formel

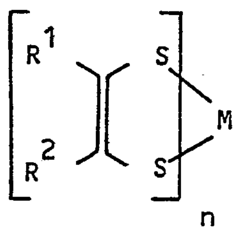

beschrieben, in der $R^1$ und $R^2$ Wasserstoff, aliphatische Reste, alicyclische Reste, insbesondere Cyclohexyl- und substituierte Cyclohexylreste oder aromatische Reste, insbesondere Phenyl- und substituierte Phenylreste, n 2 oder 3 und M ein Übergangsmetall sind. Nach den Angaben auf Seite 1, Z. 59 ff sollen die Eisen-, Nickel-, Palladium-, Platin- und Cobaltkomplexe am wirksamsten sein. Die Komplexe können nach Seite 1, Z. 72 ff auch zum Schutz von Polymeren verwendet werden. In diesem Zusammenhang wird auf Seite 2, Z. 24 ff der Nickelkomplex ($R^1 = R^2 =$ Phenyl, M=Ni und n=2) genannt.

Insbesondere die planaren, binären Metallkomplexe (I) mit Nickel, Palladium und Platin für Me, die eine intensive Absorption zwischen etwa 750 und 950 nm aufweisen, sind intensiv untersucht und als IR-Farbstoffe für optische Aufzeichnungsmedien oder Laserbauelemente und IR-Absorber für Brillengläser, Filter und andere optische Anwendungen verwendet worden.

Dithiolen-Komplexe (I), in denen $R^1$ und $R^2$ aromatische Reste sind, zeigen infolge des grossen, planaren Chromophors je nach Zentralmetallatom (Me) entweder eine grosse intermolekulare Wechselwirkung zwischen den Metallatomen und den Schwefelatomen, die zu Bänderstrukturen führen, oder eine ausgeprägte Kristallisationstendenz, wobei die Komplexe bei höheren Temperaturen nur unter Zersetzung schmelzen. Für viele Anwendungen ist eine ausreichende Löslichkeit in organischen Lösungsmitteln und/oder Bindemitteln notwendig. Diese kann häufig nur durch aufwendige Substitution des Dithiolen-Chromophors mit langkettigen Alkyl-, Phenylalkyl-, Alkoxy- und Alkylamino-Resten erreicht werden.

Aufgabe der Erfindung war es, Dithiolen-Komplexe aufzufinden, die leicht zugänglich sind und in den üblichen zur Herstellung von Aufzeichnungsmedien verwendeten Kunststoffen leicht löslich sind.

Es wurde gefunden, dass Dithiolen-Komplexe der Formel

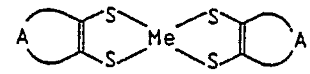

(III),

in der Me für Nickel, Palladium oder Platin und

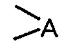 für 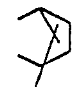

stehen, die gestellten Forderungen erfüllen.

Die neuen Dithiolen-Komplexe liegen in Form der cis- oder trans-Isomeren

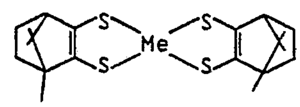

(III a)

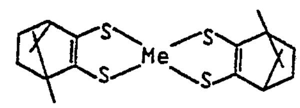

(III b)

oder in Form von Gemischen dieser Isomeren vor.

Die Dithiolen-Komplexe (III) sind in organischen Lösungsmitteln und in Kunststoffen gut bis sehr gut löslich. Ausserdem sind die neuen Komplexe im Hochvakuum leicht sublimierbar.

Aufgrund dieser Eigenschaften gibt es viele Möglichkeiten zur Anwendung von (III). (III) kann sowohl durch Aufdampfen im Vakuum wie auch nach Nass-Verfahren durch Rakeln oder Aufschleudern aus Lösungen mit oder ohne Bindemittel in Form dünner Schichten aufgebracht werden.

Je nach dem gewählten Zentralmetallatom (Me) in III können diese neuen Dithiolenkomplexe, z. B. als Stabilisatoren von Polymeren und organischen Materialien gegenüber oxidativer Schädigung (vergl. DE-A-1 941 203), als Antioxidantien und Korrosionsinhibitoren (vergl. US-A-4 427 560) oder als Schutzschicht von farbphotographischen Schichten oder Textilfärbungen (DE-A-2 456 075) verwendet werden. Auch die Verwendung als Katalysatoren erscheint sinnvoll, z. B. zur Zersetzung von Peroxiden (GB-A-1 263 910), zur Darstellung von Phenolen und Ketonen, sowie zur katalytisch-photochemischen Produktion von Wasserstoff nach dem in der US-A-4 325 793 beschriebenen Verfahren.

Die neuen IR-Farbstoffe (III) werden vorzugsweise zur Herstellung von optischen Aufzeichnungsmedien angewendet.

Die Komplexe (III) sind nach dem folgenden Reaktionsschema in guten Ausbeuten zugänglich:

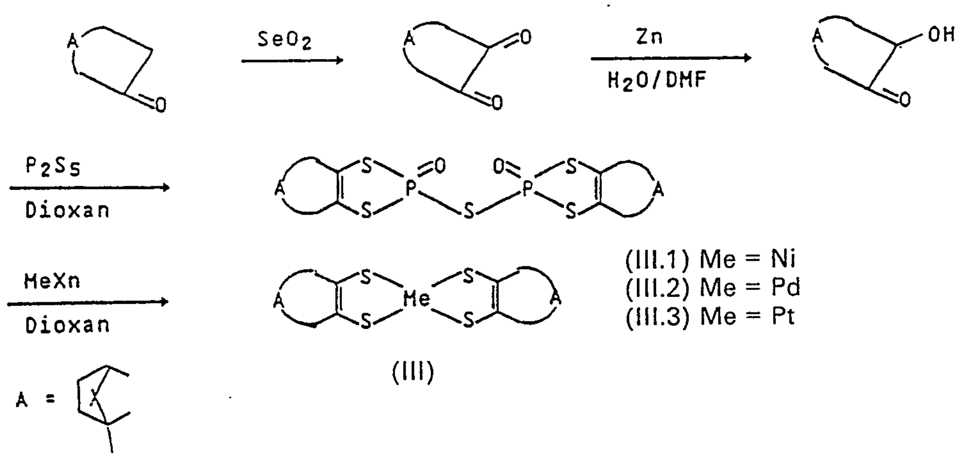


(III)

(III.1) Me = Ni
(III.2) Me = Pd
(III.3) Me = Pt

Ausgehend von optisch aktivem oder racemischem Campher erhält man nach H. RUPE und A.T. di VIGNANO, Helv. Chim. Acta *20*, S. 1078 (1937) in 90% Ausbeute Campherchinon. Dieses wird zum α-Hydroxy-campher reduziert [W. KREISER, Liebigs Ann. Chem. *745*, S. 164 (1971)]. Nach einer modifizierten Arbeitsvorschrift von G. N. Schrauzer, J. Amer. Chem. Soc. *87*, S. 1483-1489 (1965), wird das α-Hydroxyketon mit Phosphorpentasulfid in ein phosphororganisches Zwischenprodukt überführt, welches nach Abfiltrieren der anorganischen Bestandteile ohne Isolation durch Umsetzung mit dem entsprechenden Metallsalz die jeweiligen Dithiolenkomplexe (III) liefert.

Die physikalischen und spektroskopischen Daten der neuen Verbindungen (III) sind im experimentellen Teil zusammengefasst.

Wegen der Vorteile, die Festkörper-Injektionslaser bieten, sind bei optischen Aufzeichnungssystemen solche von grossem Interesse, die mit diesen Lasern beschrieben und gelesen werden können. D. h., die optischen Aufzeichnungsmedien müssen im Bereich dieser Laser, also etwa zwischen 700 und 900 nm, eine hohe Absorption haben.

Als Farbstoffe für solche optischen Aufzeichnungsmedien sind insbesondere substituierte Phthalocyanine mit verschiedenen zentralen Metallatomen, Azo-Metall-Komplexfarbstoffe mit Chrom oder Cobalt als Zentralatom und Dithiolen-Komplexe mit Nickel, Palladium oder Platin bekannt.

Die Farbstoffe wurden durch Aufdampfen oder durch Beschichten auf die Träger aufgebracht. In diesem Zusammenhang soll auf folgenden Stand der Technik hingewiesen werden: US-A-4 458 004 und 4 241 355, EP-A-13 453, 83 991 und 84 729, JP-A-67 093/1984, 82 095/1982 und 82 096/1982. Der Aufbau und die Herstellung der optischen Aufzeichnungsmedien ist bekannt (US-A-4 320 489; DE-A-2 951 341, 3 319 738 und 3 032 135).

Die neuen Dithiolen-Komplexe (III) können nach bekannten Verfahren zu optischen Aufzeichnungsmedien allein oder im Gemisch mit anderen Farbstoffen verarbeitet werden.

Vorzugsweise erfolgt das Aufbringen der die Komplexe (III) enthaltenden Schicht durch Aufrakeln, Tauchen, insbesondere durch Aufschleudern von gelöstem oder dispergiertem (III). Metallische Reflexionsschichten werden vorzugsweise durch Aufdampfen aufgebracht. Man kann auch geeignete Metallfolien aufbringen.

Für das Aufbringen der Absorptionsschichten aus Lösung bereitet man in einem geeigneten Lösungsmittel, wie Methylenchlorid, Chloroform, Tetrachlorkohlenstoff, Aceton, Methylethylketon, Cyclohexanon, Toluol, Acetonitril, Essigester, Methanol oder Mischungen dieser Lösungsmittel, eine Lösung oder gegebenenfalls eine Dispersion des Farbstoffs oder Farbstoffgemisches und des Polymeren und setzt gegebenenfalls ein Bindemittel zu.

Als Bindemittel kommen entweder durch Strahlung oder Wärme härtbare Harze, z. B. Photopolymere, Silikonharze sowie Epoxidharze oder thermoplastische Kunststoffe in Frage.

Bevorzugt werden thermoplastische Kunststoffe mit keinem oder nur sehr geringem kristallinen Anteil und Glastemperaturen von >35° C, insbesondere >75° C. Darüber hinaus müssen die Kunststoffe mit den erfindungsgemässen Thiolenverbindungen gut verträglich sein. Geeignet sind beispielsweise wasserunlösliche Bindemittel mit hohem Lösungsvermögen für die Thiolenverbindung, wie (Meth)-Acrylatpolymere und -copolymere, Polystyrol-homo und -copolymerisate, Polyvinylcarbazol, Polyvinylestercopolymere, Polyvinylchlorid und Celluloseester. Die beim Absorbieren des Laserlichtes entstehende Wärme führt zu einem radial nach aussen gerichteten Fliessen des Thermoplastes und somit zur Ausbildung von kantenscharfen «Löchern» verbunden mit einem ausgezeichneten Signal/Rausch-Verhalten der Information.

Diese Farbstoffzubereitung wird dann durch Rakeln oder Tauchen, vorzugsweise aber durch Aufschleudern auf ein vorher gereinigtes oder vorbehandeltes («subbing-layer») Substrat aufgebracht und die Schicht an der Luft getrocknet oder gehärtet. Der Film kann auch im Vakuum bei erhöhter Temperatur, oder gegebenenfalls mit Strahlung getrocknet bzw. gehärtet werden.

Je nach Systemaufbau wird zuerst die Farbstoff-in-Polymer-Schicht und dann der Reflektor aufgebracht oder umgekehrt verfahren. Auf das Aufbringen von Zwischen- und Schutzschichten oder einer reflektierenden Schicht kann gegebenenfalls verzichtet werden.

Die Erfindung soll durch die folgenden Beispiele weiter erläutert werden. Die im folgenden angegebenen Prozente beziehen sich auf das Gewicht.

*Beispiel 1*

Bis-camphenyldithio-nickel (III.1)

10,1 g (60 mmol) optisch aktiver α-Hydroxycampher, 7,9 g (60 mmol) Ammoniumsulfat und 44,4 g (0,2 mol) Phosphorpentasulfid werden in 100 ml Dioxan aufgeschlämmt und 2 Stunden am Rückfluss gekocht. Nach Abkühlen auf Raumtemperatur wird die Suspension filtriert und mit etwa 20 ml Dioxan gewaschen. Zum Filtrat wird eine Lösung von 7,2 g (30 mmol) Nickel-(II)-chlorid-Hexahydrat in 30 ml Wasser gegeben und 2 Stunden auf Rückflusstemperatur erwärmt. Die Reaktionslösung wird langsam abgekühlt, wobei ein dunkelgrüner Niederschlag ausfällt. Der Niederschlag wird abgesaugt, mit wenig kaltem Dioxan, dann mit Wasser und zuletzt mit wenig Ethanol gewaschen und im Vakuum getrocknet. Ausbeute: 10,5 g (77% d. Th.) Rohprodukt, das im DC (Kieselgel/Toluol: $R_f$=0,63) nur einen geringen Startfleck aufwies. Durch Filtration über eine kurze Kieselgel-Säule wurde analysenreines (III.1) erhalten.

Schmp.: 280-285° C

*Analyse* $C_{20}H_{28}NiS_4$ (455):

| | | | | |
|---|---|---|---|---|
| Ber.: | C 52,8 | H 6,2 | S 28,1 | Ni 13,0% |
| Gef.: | C 52,7 | H 5,9 | S 27,9 | Ni 13,0% |

UV: $\lambda_{max}$=786 nm, ε=23 530 (Methylenchlorid);
$\lambda_{max}$=794 nm, ε=23 580 (Toluol).

IR (KBr): ν= 2958, 1342, 1282, 1247, 1204, 1182, 1160, 1107, 1071, 684 $cm^{-1}$.

$^1$H-NMR ($CDCl_3$): δ= 0,65 s [6H], 0,95 s [6H], 1,25 d [4H], 1,40 s [6H], 1,82 t [2H], 2,08 d (t) [2H], 3,12 d [2H].

$^{13}$C-NMR ($CDCl_3$): δ= 11,5, 20,3 (2C), 26,0, 33,0, 59,9, 61,4, 62,0, 193,7, 198,5.

MS (70 eV): m/e = 454 (100%) $M^{\oplus}$ [$Ni^{58}$]+ Ni-Isotopenmuster; 411 (60%) $M^{\oplus}$[$Ni^{58}$]$-C_3H_7$ +Ni-Isotopenmuster.

*Beispiel 2*

Bis-camphenyldithio-palladium (III.2)

5,05 g (30 mmol) optisch aktiver α-Hydroxycampher, 4,0 g (30 mmol) Ammoniumsulfat und 9,8 g (44 mmol) Phosphorpentasulfid werden in 80 ml Dioxan aufgeschlämmt und 2 Stunden am Rückfluss gekocht. Nach Abkühlen auf Raumtemperatur wird die Suspension filtriert und mit 20 ml Dioxan gewaschen. Zum Filtrat wird eine Lösung in 1,63 g (5 mmol) Kaliumtetrachloropalladat-(II) in 32,5 ml Wasser gegeben und erneut 2 Stunden auf Rückflusstemperatur gehalten. Die Reaktionsmischung wird abgekühlt, mit 10 ml kaltem Dioxan gewaschen, in möglichst wenig Methylenchlorid aufgenommen und über eine 10 cm lange Säule (Kieselgel 60) filtriert. Die violette Fraktion lieferte 2,2 g (88% d. Th.) analysenreinen Palladiumkomplex (III.2).

Schmp.: 278-281° C

*Analyse* $C_{20}H_{28}PdS_4$ (502):

Ber.: Pd 21,1%

Gef.: Pd 19,8%

UV: $\lambda_{max}$=830 nm, ε=28 322 (Methylenchlorid); 833 nm, ε=27 663 (Toluol); 834 nm, ε= 19 559 (Dimethylformamid).

IR (KBr): ν=2956, 1339, 1292, 1285, 1247, 1207, 1182, 1160, 1107, 1073, 687, 564, 433 $cm^{-1}$.

$^1$H-NMR ($CDCl_3$): δ=0,78 s [3H], 0,82 s [3H], 0,97 s [6H], 1,34 s [6H], 1,34-1,45 m [4H], 1,87 t [1H], 1,88 t [1H], 2,15 m [2H], 2,98 s [1H], 3,01 s [1H].

$^{13}$C-NMR ($CDCl_3$): δ=11,15, 20,35, 20,44, 26,73, 33,78, 60,80, 61,20, 61,36, 62,66, 195,72, 195,98, 200,36, 200,48.

MS (70 eV): m/e=502 (100%) $M^{\oplus}$ [$Pd^{106}$]+ Isotopenmuster; 459 $M^{\oplus}$ [$Pd^{106}$]$-C_3H_7$+Isotopenmuster.

*Beispiel 3*

Bis-camphenyldithio-platin (III.3)

4,8 g (28,8 mmol) optisch aktiver α-Hydroxycampher, 3,8 g (28,8 mmol) Ammoniumsulfat und 9,8 g (44 mmol) Phosphorpentasulfid werden in 100 ml Dioxan aufgeschlämmt und 2 Stunden am Rückfluss gekocht. Nach Abkühlen auf Raumtemperatur wird die Suspension filtriert und mit etwa 20 ml Dioxan gewaschen. Dem Filtrat wird eine Lösung von 2,0 g (4,8 mmol) Kaliumtetrachloroplatinat-(II) in 33 ml Wasser zugegeben und erneut 2 Stunden bei Rückflusstemperatur gehalten. Die Reaktionsmischung wird langsam abgekühlt, wobei ein feinkristalliner violetter Niederschlag ausfällt, die isolierte Fällung wird mit wenig kaltem Dioxan, dann Wasser und zuletzt Ethanol gewaschen und dann im Vakuum getrocknet. Das Rohprodukt (2,5 g, 88% d. Th.) weist im DC (Kieselgel/Toluol: $R_f$=0,63, Methylenchlorid: $R_f$=0,92) einen kleinen Startfleck auf. Durch Filtrieren der Lösung in Methylenchlorid über eine kurze Säule mit Kieselgel wurde (III.3) in reiner Form erhalten. Ausbeute: 2,3 g (81% d. Th.).

Schmp.: 301-302° C

*Analyse* $C_8H_{28}PtS_4$ (591):

| | | | | |
|---|---|---|---|---|
| Ber.: | C 40,6 | H 4,7 | S 21,7 | Pt 33,0% |
| Gef.: | C 40,8 | H 4,9 | S 21,6 | Pt 34,3% |

UV: $\lambda_{max}$=776 nm (Methylenchlorid); 768 nm, ε=45 030 (Toluol).

IR (KBr): ν=2957, 1344, 1293, 1285, 1248, 1207, 1182, 1161, 1109, 1071, 689 $cm^{-1}$.

$^{13}$C-NMR ($CDCl_3$): δ=11,55, 20,08, 21,01, 26,33, 26,40, 33,31, 33,36, 59,10, 59,46, 59,81, 61,11, 61,43, 61,60, 61,67, 191,13, 191,39, 195,98, 196,07.

MS (70 eV): m/e=590, 591 (100%), 592, 593, 595 M$^{\oplus}$ (Isotopenpeaks), 547, 548, 549, 550 M$^{\oplus}$ (Isotopenpeaks)$-C_3H_7$.

*Beispiel 4*

Bis-camphenyldithio-nickel (III.1)

Ausgehend von 10,1 g (60 mmol) racemischem α-Hydroxycampher, 7,9 g (60 mmol) Ammoniumsulfat, 44,4 g (0,2 mol) Phosphorpentasulfid und 7,2 g (30 mmol) Nickel-(II)-chlorid-Hexahydrat wurden unter den gleichen Reaktions- und Aufarbeitungsbedingungen wie in Beispiel 1 4,3 g (31% d. Th.) Nickel-Komplex (III.1) erhalten, der wie folgt charakterisiert wurde:

DC: Kieselgel/Toluol $R_f$=0,62.

Schmp.: 270-276° C

UV: $\lambda_{max}$=786 nm, ε=23 010 (Methylenchlorid).

IR (KBr): ν=2958, 1343, 1292, 1285, 1249, 1257, 1181, 1161, 1108, 1070, 730, 562, 440 $cm^{-1}$.

$^1H$-NMR ($CDCl_3$): δ=0,65 s [6H], 0,96 [6H], 1,24 s [2H], 1,28 s [2H], 1,42 s [6H], 1,83 t [2H], 2,09 d (t) [2H], 3,09 d [2H].

$^{13}C$-NMR ($CDCl_3$): δ=11,47, 20,25 (2C), 25,97, 33,01, 59,85, 61,35, 62,00, 193,65, 198,41.

MS (70 eV): m/e=454 (100%) M$^{\oplus}$ [$Ni^{58}$]$-$H +Isotopenmuster; 411 (55%) M$^{\oplus}$ [$Ni^{58}$]$-C_3H_7$ +Isotopenmuster.

*Beispiel 5*

Bis-camphenyldithio-palladium (III.2)

8,4 g (50 mmol) racemischer α-Hydroxycampher, 6,6 g (50 mmol) Ammoniumsulfat und 17 g (75 mmol) Phosphorpentasulfid werden in 75 ml Dioxan aufgeschlämmt und 2 Stunden am Rückfluss gekocht. Nach Abkühlen auf Raumtemperatur wird die Suspension filtriert und mit 20 ml Dioxan gewaschen. Zum Filtrat wird eine Lösung von 33 g (10 mmol) Kaliumtetrachloropalladat-(II) in 50 ml Wasser gegeben und erneut 2 Stunden auf Rückflusstemperatur gehalten. Die Reaktionsmischung wird abgekühlt, das ölige, teilweise kristalline Produkt abfiltriert, in Methylenchlorid aufgenommen, mit Wasser gewaschen und getrocknet. Das Rohprodukt wird in 25 ml Toluol gelöst und über eine 50-cm-Säule an Aluminiumoxid chromatographiert. Die violette Fraktion liefert 3,6 g (72% d. Th.) (III.2).

Schmp.: 275-277° C (nach Subl.)

*Analyse* $C_{20}H_{28}PdS_4$ (502):

Ber.: Pd 21,2%

Gef.: Pd 19,9%

UV: $\lambda_{max}$=836 nm (Methylenchlorid); 823,5 (Methanol); 837 nm, ε=20 860 (Toluol).

*Beispiel 6*

Bis-camphenyldithio-platin (III.3)

Ausgehend von 4,8 g (29 mmol) racemischem α-Hydroxycampher, 3,8 g (29 mmol) Ammoniumsulfat, 9,8 g (44 mmol) Phosphorpentasulfid und 2,0 g (4,8 mmol) Kaliumtetrachloroplatinat-(II) wurde wie in Beispiel 3 gearbeitet. Ausbeute: 1,9 g (67% d. Th.) Platin-Komplex (III.3), der wie folgt charakterisiert wurde:

DC: Kieselgel/Methylenchlorid $R_f$=0,92.

Schmp.: 265-275° C

UV: $\lambda_{max}$=769 nm, ε=39 810 (Toluol).

IR (KBr): ν=2957, 1344, 1293, 1285, 1249, 1207, 1182, 1161, 1109, 1070, 681, 560, 430 $cm^{-1}$.

$^1H$-NMR ($CDCl_3$): δ=0,71 s [3H], 0,73 s [3H], 0,96 s [6H], 1,25-1,40 m [4H], 1,40 s [6H], 1,80 t [2H], 1,98-2,12 m [2H], 3,06 d [2H].

MS (70 eV): m/e=590, 591 (100%), 592, 593, 594 M$^{\oplus}$ (Isotopenmuster), 547 (60%)+Isotopenmuster.

*Anwendungsbeispiel 1*

Eine 1,2 mm dicke Polymethylmethacrylatscheibe mit einem Durchmesser von 120 mm und einem Innenlochdurchmesser von 15 mm wird vorgereinigt (Entfernung von Staubpartikeln) und unter Reinraumbedingungen mit einer etwa 0,3 µm dicken Glättungsschicht aus hochmolekularem Polymethylmethacrylat versehen. Auf die Glättungsschicht wird eine Lösung von 1 g des Farbstoffs (III.3) 2 und 1 g eines Copolymeren aus Methacrylat/Methacrylsäure (70:30) in Chloroform nach dem Schleuderverfahren bei 4800 U/min auf den Träger aufgetragen, wobei das Lösungsmittel abdunstet und eine stabile, ca. 0,3 µm dicke Farbstoff enthaltende Polymerschicht gebildet wird. In einer Vakuumaufdampfapparatur wird ein Aluminiumspiegel mit einer Schichtdicke von 0,03 µm auf die Farbstoffschicht aufgebracht und darauf eine Schutzschicht von 1,2 µm Dicke aus Polystyrol in Xylol aufgeschleudert.

Zwei derartige Platten werden über geeignete Abstandsringe sandwichartig mit den beschichteten Seiten nach innen zusammengeklebt, so dass zwischen den Platten ein Luftspalt von etwa 0,4 mm verbleibt. Sowohl in die einzelnen Platten als auch in das Sandwich-Aufzeichnungsmedium werden mit einem justierten AlGaAs-Laser einzelne, etwa 1 µm grosse Löcher in die sensitive Schicht geschrieben. Diese Loch-Information lässt sich mit Halbleiterlaserlicht wieder auslesen. Die beste Empfindlichkeit und ein sehr gutes Signal-zu-Rauschen-Verhältnis werden mit Laserlicht der Wellenlänge λ=780 nm erreicht.

*Anwendungsbeispiel 2*

Es wird wie in Anwendungsbeispiel 1 vorgegangen, jedoch eine Lösung von 0,045 g des Farbstoffs (III.1), 0,05 g des Farbstoffs (III.2), 0,06 g des Farbstoffs (III.3) und 0,2 g Polymethylmethacrylat in 6 ml Methylenchlorid zum Aufbringen der Dye-in-polymer-Schicht verwendet.

*Anwendungsbeispiel 3*

Es wird wie in Anwendungsbeispiel 1 vorgegangen, jedoch wird die laserlichtempfindliche Schicht durch Verdampfen von III.3 im Vakuum

aus einem Tantalschiffchen (T=200° C, p≦$10^4$ Torr) erzeugt. Dicke der Schicht: ca. 300 Å.

## Patentansprüche

1. Dithiolen-Komplexe der Formel

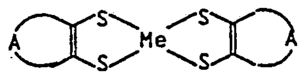

in der Me für Nickel, Palladium oder Platin und

>A für 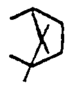

stehen.

2. Dithiolen-Komplexe gemäss Anspruch 1, *dadurch gekennzeichnet*, dass Me für Nickel steht.

3. Dithiolen-Komplexe gemäss Anspruch 1, *dadurch gekennzeichnet*, dass Me für Palladium steht.

4. Dithiolen-Komplexe gemäss Anspruch 1, *dadurch gekennzeichnet*, dass Me für Platin steht.

5. Verwendung der Dithiolen-Komplexe gemäss Anspruch 1 als Stabilisatoren.

6. Verwendung der Dithiolen-Komplexe gemäss Anspruch 1 als Antioxidantien.

7. Verwendung der Dithiolen-Komplexe gemäss Anspruch 1 als Korrosionsinhibitoren.

8. Verwendung der Dithiolen-Komplexe gemäss Anspruch 1 als Katalysatoren.

9. Verwendung der Dithiolen-Komplexe gemäss Anspruch 1 in optischen Aufzeichnungsmedien.

## Claims

1. A dithiolene complex of the formula

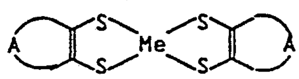

where Me is nickel, palladium or platinum, and

>A is 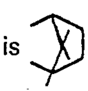

2. A dithiolene complex as claimed in claim 1, wherein Me is nickel.

3. A dithiolene complex as claimed in claim 1, wherein Me is palladium.

4. A dithiolene complex as claimed in claim 1, wherein Me is platinum.

5. The use of a dithiolene complex as claimed in claim 1 as a stabilizer.

6. The use of a dithiolene complex as claimed in claim 1 as an antioxidant.

7. The use of a dithiolene complex as claimed in claim 1 as a corrosion inhibitor.

8. The use of a dithiolene complex as claimed in claim 1 as a catalyst.

9. The use of a dithiolene complex as claimed in claim 1 in optical recording media.

## Revendications

1. Complexes de dithiolène de formule

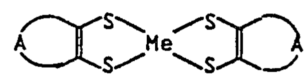

dans laquelle Me est mis pour un atome de nickel, de palladium ou de platine et

>A est mis pour 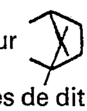

2. Complexes de dithiolène selon la revendication 1, caractérisés en ce que Me est mis pour un atome de nickel.

3. Complexes de dithiolène selon la revendication 1, caractérisés en ce que Me est mis pour un atome de palladium.

4. Complexes de dithiolène selon la revendication 1, caractérisés en ce que Me est mis pour un atome de platine.

5. Utilisation des complexes de dithiolène selon la revendication 1 comme stabilisants.

6. Utilisation des complexes de dithiolène selon la revendication 1 comme antioxydants.

7. Utilisation des complexes de dithiolène selon la revendication 1 comme inhibiteurs de corrosion.

8. Utilisation des complexes de dithiolène selon la revendication 1 comme catalyseurs.

9. Utilisation des complexes de dithiolène selon la revendication 1 dans des supports d'affichage optique.